# EUROPEAN PATENT SPECIFICATION

(11) **EP 2 189 790 B1**
(45) Date of publication and mention of the grant of the patent: **20.07.2016**
(21) Application number: 08827849.4
(22) Date of filing: 20.08.2008
(51) Int. Cl.: G01N 33/48, G01N 33/15, G01N 33/50, G01N 33/68

(54) **METHOD FOR QUANTIFICATION OF TITER OF NEUROTOXIN-NEUTRALIZING ANTIBODY**
VERFAHREN ZUR QUANTIFIZIERUNG DES TITERS NEUROTOXIN-NEUTRALISIERENDER ANTIKÖRPER
PROCÉDÉ DE QUANTIFICATION DE TITRE D'UN ANTICORPS NEUTRALISANT UNE NEUROTOXINE

(30) Priority: 20.08.2007 JP 2007213889
(43) Date of publication of application: 26.05.2010
(73) Proprietor: The Chemo-Sero-Therapeutic Research Institute, Kumamoto-shi Kumamoto 860-8568 (JP); JAPAN as represented by DIRECTOR GENERAL OF NATIONAL INSTITUTE OF INFECTIOUS DISEASES, Tokyo 162-8640 (JP)
(72) Inventor: TORII, Yasushi, Kumamoto-shi Kumamoto 860-8568 (JP); HARAKAWA, Tetsuhiro, Kumamoto-shi Kumamoto 860-8568 (JP); GOTO, Yoshitaka, Kumamoto-shi Kumamoto 860-8568 (JP); GINNAGA, Akihiro, Kumamoto-shi Kumamoto 860-8568 (JP); KAJI, Ryuji, Tokushima-shi Tokushima 770-8503 (JP); KOZAKI, Shunji, Sakai-shi Osaka 599-8531 (JP); TAKAHASHI, Motohide, Musashimurayama-shi Tokyo 208-0011 (JP)
(74) Representative: Vossius & Partner Patentanwälte Rechtsanwälte mbB
(86) International application number: PCT/JP2008/064813
(87) International publication number: WO 2009/025290

(56) References cited:
- CICHON J V ET AL: "The effect of botulinum toxin type A injection on compound muscle action potential in an in vivo rat model" LYRYNGOSCOPE, vol. 105, no. 2, 1995, pages 144-148, XP008104222
- SESARDIC D ET AL: "Detection of antibodies against botulinum toxins." MOVEMENT DISORDERS, vol. 19 Suppl 8, 2004, pages S85-S91, XP002591670
- TORII Y ET AL: "Quantification of potency of neutralizing antibodies to botulinum toxin using compound muscle action potential (CMAP)." TOXICON, vol. 55, no. 2-3, February 2010 (2010-02), pages 662-665, XP026822393
- TORII Y ET AL: "Quantification of potency of neutralizing antibody to botulinum toxin by measuring the compound muscle action potential (CMAP)" TOXICON, vol. 51, no. Suppl. 1, 62, June 2008 (2008-06), page 21, XP002591671
- GORDON P H ET AL: "Extensor digitorum brevis test and resistance to botulinum toxin type A." MUSCLE AND NERVE, vol. 26, no. 6, 2002, pages 828-831, XP002591672
- KESSLER K R ET AL: "The EDB test - A clinical test for the detection of antibodies to botulinum toxin type A" MOVEMENT DISORDERS, vol. 12, no. 1, 1997, pages 95-99, XP002591673
- HALL Y.H.J. ET AL.: 'Novel application of an in vitro technique to the detection and quantification of botulinum neurotoxin antibodies' J.IMMUNOL.METHODS vol. 288, no. 2, 2004, pages 55 - 60, XP008131631
- MOTOHIDE TAKAHASHI ET AL.: 'Fukugokin katsudo den'i ni yoru botulinus shinkei dokuso no kinshikan kassei no teiryoka' JAPANESE JOURNAL OF CLINICAL NEUROPHYSIOLOGY vol. 34, no. 5, 2006, page 413,P1-A-9-4
- PEARCE L.B. ET AL.: 'Measurement of Botulinum Toxin Activity: Evaluation of the Lethality Assay' TOXICOL.APPL.PHARMACOL. vol. 128, no. 1, 1994, pages 69 - 77, XP008131630
- OSAMU MIMURA ET AL.: 'Botulinus ryoho no genjo to shorai no tenbo' BRAIN 21 vol. 5, no. 4, 2002, pages 416 - 419

## Description

### TECHNICAL FIELD

The present invention relates to a method for quantifying a titer of a neutralizing antibody to a neurotoxin. Specifically, the present invention relates to a method for quantifying a titer of a neutralizing antibody to a neurotoxin that has a muscle relaxing activity to a mammal and is produced by bacteria of Clostridium. More specifically, the present invention relates to a method for quantifying a titer of a neutralizing antibody to a neurotoxin characterized by that a mixture of a neurotoxin and a neutralizing antibody is administered to a mammal and an extent of muscular relaxation in a mammal by a neurotoxin not neutralized is quantified using an electromyograph.

### BACKGROUND ART

There is known a neurotoxin including one produced by bacteria of Clostridium or by several fish or shellfish, typically a swellfish poison tetrodotoxin, a snake toxin alpha-bungarotoxin, and the like. These toxins, though different in their point of action, commonly block neurotransmission at the nerve end to thereby exhibit a muscular relaxation in a mammal to which the toxins are inoculated. Among these, Clostridium toxin is a neurotoxin produced by bacteria of Clostridium which is divided into more than a hundred groups based on the form and function. For bacteria of Clostridium, Clostridium baratii, Clostridium butyricum, Clostridium botulinum, Clostridium tetani, and the like are known. A botulinum toxin produced by Clostridium botulinum, anaerobic Gram-positive bacteria, is the most lethal neurotoxin on earth. It is classified into seven types, A, B, C, D, E, F and G, and the property of each type has been elucidated. The types are distinguishable from each other by respective type-specific neutralizing antibodies. Depending on the types, a botulinum toxin may vary in animal species it may affect, severity of paralysis it induces, duration of time of its action, and the like (Non-patent reference 1).

When botulinus or botulinum toxin enters human being, "botulism" occurs. Most of botulism is caused through intake of food contaminated with botulinus that produces botulinum toxin (food botulism). Although botulinus, when orally taken, does not survive due to gastric acid or enterobacterium, it survives under exceptional conditions to reach the intestine tracts where it proliferates to cause intoxication (infant botulism). Botulism is also caused from unclean wound where botulinum proliferates (wound botulism). There is also "non-classifiable botulism" which is classified into neither of the three botulisms (Non-patent reference 1). For therapeutically treating botulism, sufficient respiratory management and administration of antiserum are conducted. Botulinum antiserum is commercially available as dry botulinum equine antiserum (types A, B, E and F) from Juridical Foundation The Chemo-Sero-Therapeutic Research Institute.

On the other hand, although a botulinum toxin is a neurotoxin that may lead human to death in botulism through blockage of systemic neurotransmission, it may also be utilized as a remedy for treating a disease with an accelerated muscular tension such as e.g. dystonia by positively making use of its activity and by administering directly into the muscle of patients suffering from the disease so that a local muscular tension may be relieved. For instance, a type A botulinum toxin complex (BOTOX; registered trademark) has been approved as a medicament for treating blepharospasm, strabismus, hemifacial spasm, and cervical dystonia, and for treating wrinkles at the middle of the forehead by the Food and Drug Administration (FDA). A type B botulinum toxin complex (MYOBLOC; registered trademark) has also been approved as a medicament for treating cervical dystonia by FDA. It is said that a type A botulinum toxin has a higher potency and a longer duration of action as compared to types other than a type A botulinum toxin. A type A botulinum toxin in peripheral muscles may usually exert a pharmacological activity within 24 hours after injection but clinical efficacy may often be observed two to three days after injection. An average duration of action of a type A botulinum toxin from its single muscular administration up till amelioration of symptoms is typically about 3 to 4 months.

However, when a botulinum toxin is used as a medicament, a problem of antibody production may arise since a botulinum toxin is an exogenous protein to the human body. The most problematic here is a neutralizing antibody. Upon production of a neutralizing antibody, efficacy may be expected to some extent by increasing a dose when its titer is low but, when the titer becomes elevated, efficacy of a botulinum toxin may be lost (Non-patent references 2 and 3). Thus, when a botulinum toxin is used as a medicament, antibody production is a serious issue since therapeutic efficacy by a botulinum toxin may be lost when a titer of a neutralizing antibody is elevated. It is thus very important to find production of a neutralizing antibody at an early stage and to control a dose and administration schedule of a botulinum toxin at therapy.

There are several methods for quantification of an anti-toxin antibody with varying sensitivity (Non-patent reference 4). Exemplary methods for quantification of an anti-toxin antibody include the followings:

### (1) Mouse neutralization assay (Mouse Lethality Assay: MLA)

An antibody titer may be measured by this approach wherein a mixture of a neurotoxin and a neutralizing antibody thereto is administered to mice and the number of mice which died from the neurotoxin not neutralized is counted with an approximate sensitivity being 10 mIU/mL (Non-patent reference 5). This is the oldest approach for measuring a titer of a neutralizing antibody.

### (2) Mouse Diaphragm Assay:MDA

An antibody titer may be estimated by this approach wherein a mouse phrenic nerve/diaphragm specimen is prepared and effect of a toxin to said specimen is observed. Its detection sensitivity of a neutralizing antibody is around 0.3 mIU/mL, which is higher than that of MLA (Non-patent reference 5).

### (3) In vitro quantification

In vitro method for quantification includes Western blot which however is low both in sensitivity and specificity (Non-patent reference 7). Additional in vitro methods include hapten-labeled elution, immunoprecipitation assay (IPA), ELISA (enzyme-linked immunosorbent assay), and the like (Non-patent reference 4) wherein sensitivity is as high as 0.3 to 1.0 mIU/mL but no discrimination is possible between neutralizing and non-neutralizing antibodies. Another in vitro method for quantification of a neutralizing antibody is an endopeptidase assay wherein cleaved products of SNAP-25 are detected with an anti-SNAP-25 antibody, which assay is proved to have a higher sensitivity than MLA (Non-patent reference 8). In vitro quantification method the most highly estimated at present is IPA. IPA or MDA has a higher sensitivity among MLA, MDA and IPA. Specificity is the highest in MLA. IPA does not show 100% specificity but has advantages of not killing animals, and of being simple, speedy and cheap.

MLA is eligibly used even nowadays for measurement of a titer of a neutralizing antibody to a neurotoxin such as botulinum toxin. This approach, with an index of a lethal activity of the remaining toxin not neutralized in mice, is however reported to have a sensitivity which is low and extremely varies among laboratories (Non-patent reference 9). Besides, using a large number of mice, this approach is problematic from viewpoints of ethic and animal protection. On the other hand, among patients receiving therapy with a botulinum toxin are those who, though in a rare case, do not show therapeutic efficacy and have a trace amount of antibodies in their serum. The detection sensitivity of the MLA method however cannot measure these antibodies and hence a measurement system with a higher sensitivity is desired (Non-patent reference 1).

A method using an electromyograph is also reported for detection of the presence or absence of a neutralizing antibody to a neurotoxin. This is an EDB test wherein a small amount of a botulinum toxin is intramuscularly administered to such muscles in patients as one whose paralysis would not interfere with daily life, typically extensor digitorum brevis; EDB), and CMAP of the same muscle is measured and compared before and 1-2 weeks after the administration. Its sensitivity is estimated to be around 1 mIU/mL. However, this method is disadvantageous in that a neurotoxin must be administered to the unnecessary muscles in patients and that it takes too much time until results are obtained (Non-patent reference 4).
Non-patent reference 1: Ryuji Kaji et al., "Dystonia and botulinum therapy", Shindan-To-Chiryosha, 2005
Non-patent reference 2: Dressler et al., Eur. Neurol., 2002, 47, p.118-121
Non-patent reference 3: Dressler et al., Eur. Neurol., 2002, 48, p.26-29
Non-patent reference 4: Sesaardic et al., Mov. Disord., 2004, 19(Suppl 8), p.S85-S91
Non-patent reference 5: Hatheway et al., J. Infect. Dis., 1984, 150, p.407-412
Non-patent reference 6: Goschel et al., Exp. Neurol., 1997, 147, p.96-102
Non-patent reference 7: Duane et al., Neurology, 1997, 48(Suppl2), A399
Non-patent reference 8: Hallis et al., J. Clin. Microbiol., 1996, 34, p.1934-1938
Non-patent reference 9: Sheridan et al., J. Appl. Toxicol., 1999, 19(Suppl1), p.S29-S33

### DISCLOSURE OF THE INVENTION

### (Technical Problem to be Solved by the Invention)

An object of the present invention is to provide a method for quantitatively measuring a titer of a neutralizing antibody to a Clostridium toxin with specificity and a high sensitivity.

### (Means for Solving the Problems)

The present inventors have established a method for quantification of a titer of a neutralizing antibody to a neurotoxin. In accordance with the method for measuring a neutralizing antibody using an electromyograph of the present invention, a neutralizing antibody and a toxin is reacted with each other and a titer of the neutralizing antibody is measured based on a muscular potential that is decreased by the remaining amount of the toxin not neutralized. The method of the present invention is hereinafter referred to as "neutralization CMAP method". This method is more sensitive than MLA and may detect a neutralizing antibody up to at around 1 mIU/mL. While with MLA, observation for four days after administration is necessary, the method of the present invention allows for quantification in one day after administration and hence results may be obtained rapidly. Besides, the method of the present invention is simple and highly reproducible as being the same as ordinary electromyogram. In addition, the most advantageous aspect of the method of the present invention is that no animals are killed unlike MLA in which measurement is based on lethality.

The present invention includes the following (1) to (4):
(1) A method for quantification of a titer of a neutralizing antibody to a neurotoxin comprising the following steps:
   (a) mixing a standard sample containing a fixed amount of a neurotoxin and a test sample containing a neutralizing antibody to said neurotoxin;
   (b) administering the mixture obtained in step (a) to the muscle of a non-human mammal;
   (c) several hours to several days after the administration, applying electric stimulus to the nerve which is dominant in the muscle that received the administration ;
   (d) measuring a compound muscle action potential (CMAP) due to contraction of the muscle of said mammal by application of electric stimulus with an electromyograph; and
   (e) analyzing CMAP amplitude data obtained in step (d) for an extent of decrease in amplitude by the neurotoxin not neutralized, based on a dose dependent relationship between a titer of a neutralizing antibody and CMAP amplitude, to thereby quantify a titer of the neutralizing antibody contained in the test sample.
(2) The method for quantification of (1) above wherein the neurotoxin is selected from a neurotoxin produced by bacterium of Clostridium, a neurotoxin produced by fish or shellfish and a snake toxin.
(3) The method for quantification of (2) above wherein the bacterium of Clostridium is Clostridium botulinum.
(4) The method for quantification of (3) above wherein said Clostridium botulinum is selected from type A, B, C, D, E, F and G of Clostridium botulinum.

### (More Efficacious Effects than Prior Art)

By utilizing the method of the present invention, (1) quantification of a neutralizing antibody to a neurotoxin, (2) quantification of antiserum to various types of a botulinum toxin, and (3) measurement of a titer of a neutralizing antibody in serum from patients who receive therapy with a botulinum toxin but do not show a response thereto, become possible.

In accordance with the present invention, no surgical incision or sacrifice may be done to a mammal such as rat to which a botulinum toxin is administered. By subjecting a single mammal bred continually to measurement with lapse of time, the method of the present invention allows for a long-term evaluation and is a highly accurate quantification system with less variance by days when the measurement is performed or less individual variance. Besides, the method of the present invention requires a smaller number of mammals and hence is advantageous from viewpoints of ethic and animal protection. In addition, with higher sensitivity than the conventional MLA, the method of the present invention makes it possible to measure a titer of a neutralizing antibody in patients who do not respond to therapy with a botulinum toxin.

### BRIEF DESCRIPTION OF DRAWINGS

Fig. 1 illustrates an administration site of a neurotoxin and sites for measurement of CMAP.
Fig. 2 shows comparison between titers of standard type A botulinum antitoxin and of rabbit antiserum using a neutralization CMAP method.
Fig. 3 shows a straight line of a dose response of standard type B botulinum antitoxin by a neutralization CMAP method.
Fig. 4 shows a straight line of a dose response of standard type E botulinum antitoxin by a neutralization CMAP method.
Fig. 5 shows a straight line of a dose response of standard type F botulinum antitoxin by a neutralization CMAP method.
Fig. 6 shows a calibration line of a dose response of standard type A botulinum antitoxin by a neutralization CMAP method.

### BEST MODE FOR CARRYING OUT THE INVENTION

The method for quantitatively measuring a titer of a neutralizing antibody to a neurotoxin according to the present invention may be performed by monitoring a compound muscle action potential of the hind leg muscle, preferably the gastrocnemius muscle, by electric stimulus using an electromyograph. The present invention is based on the finding that, taking into particular consideration amplitude data among electromyogram parameters monitored with an electromyograph, a titer of a neutralizing antibody may accurately be quantified by analyzing a decrease in the amplitude by the neurotoxin not neutralized.

In a broad embodiment of the present invention, for quantifying a titer of a neutralizing antibody to a neurotoxin, a neurotoxin and a neutralizing antibody to the neurotoxin is mixed, the mixture is administered to the muscle of a non-human mammal, and a compound muscle action potential at the administration site is monitored with an electromyograph for measuring an extent of muscular relaxation in the non-human mammal by the neurotoxin not neutralized. In this regard, taking into particular consideration amplitude data, analysis of an extent of decrease in amplitude by a neurotoxin not neutralized allows for quantification with high accuracy of a titer of a neutralizing antibody to a neurotoxin.

In one embodiment, the present invention provides a method for determining a titer of a neutralizing antibody to a neurotoxin.

The term "non-human mammal" as used herein includes, for instance, monkey, rat, rabbit, guinea pig, hamster, cat, mouse and dog.

A neurotoxin may be selected from those derived from bacteria of Clostridium such as Clostridium baratii, Clostridium butyricum, Clostridium tetani and Clostridium botulinum or a tetanus toxin. A botulinum toxin may be selected from types A, B, C, D, E, F and G, and a mixture thereof, and typically type A botulinum toxin.

Difference between the maximum and the minimum potentials obtained by electric stimulus applied to the muscle with an electromyograph is CMAP amplitude. This CMAP amplitude is decreased by a neurotoxin in a dose dependent manner as described in WO2007/125604 (PCT/JP2006/309040). A neutralization CMAP method applies this principle to measurement of a titer of a neutralizing antibody. An electromyograph may be one commercially available from Nicolet Biomedical (Nicolet Biking Quest series) as a medical instrument for therapy and diagnosis.

In accordance with the method of the present invention, the remaining amount of a toxin may vary with a fixed amount of a toxin and a varied titer of a neutralizing antibody wherein the more the remaining amount of a toxin is present, the more the CMAP amplitude is decreased, and the higher a titer of a neutralizing antibody is, the more a toxin is neutralized to decrease the remaining amount of a toxin, resulting in increase in the CMAP amplitude. There is a dose dependent relationship between a titer of a neutralizing antibody and CMAP amplitude which may be graphed to prepare a calibration line.

A site for administration and sites for measurement in the neutralization CMAP method are not particularly limited but, for convenience of measurement, are preferably legs, most preferably hind legs.

The neutralization CMAP method may be carried out as outlined below. A reaction solution of a neutralizing antibody and a neurotoxin is administered into the muscle. Several hours to several days after the administration, electric stimulus is applied to the nerve dominant into the muscle that received administration and CMAP potentials for said muscle are recorded using a recording electrode. CMAP amplitude is then calculated from the obtained CMAP potentials. Data from several groups are statistically analyzed with each group being administered with the same titer of a neutralizing antibody to thereby obtain a calibration line. The same procedures are repeated for a sample of an unknown titer of a neutralizing antibody and the obtained amplitude is applied to a calibration scheme to give a titer of the neutralizing antibody.

The present invention is explained in more detail by means of the following Examples but is not limited thereto.

### Preparation: Purification of botulinum neurotoxin

Botulinum types A, B, E and F neurotoxin (NTX) were purified as described in "Sakaguchi, G., Ohishi, I., and Kozaki, S., 1981, BIOCHEMICAL ASPECTS of botulism: Purification and oral toxicities of Clostridium botulinum progenitor toxins, pp 21-34, Lewis, G. E.(ed.), Academic Press, New York".

The botulinum M toxin was dialyzed against 10 mM phosphate buffer (pH 7.5), adsorbed to DEAE Sepharose column equilibrated with the same buffer, and eluted with 0 to 0.3 M NaCl gradient of the same buffer to separate the neurotoxin from a non-toxin protein. The obtained neurotoxin (NTX) was concentrated with YM-10 membrane (Millipore) to 1 mg/mL, dialyzed against 50 mM phosphate buffer (pH 7.5) and stored at -80°C till use.

### Comparative Example: Quantification of neutralizing antibody to botulinum neurotoxin by MLA method

Standard type A botulinum antitoxin (given from National Institute of Infectious Diseases) was diluted stepwise in 50 mM phosphate buffer (pH 6.0) containing 0.5 w/v% gelatin to 0.078, 0.063, 0.05, 0.04 and 0.032 IU/0.25mL. Samples were also diluted stepwise so as to be the same potency as standard type A botulinum antitoxin from estimated potency. Using the type A NTX prepared in Preparation as a test toxin, a solution containing 1 test toxin dose (a dose of a toxin with which a half of mice administered with the toxin after reaction with 0.05 IU/0.25mL die) in 0.25 mL was prepared. The antitoxin and the test toxin were mixed together at an equivalent amount for reaction for 1 hour. The reaction mixture was administered to mice intraperitoneally at 0.5 mL per mouse and the mice were observed for 4 days.

Rabbit antiserum obtained by immunization with type A botulinum toxin was tested twice by MLA method to show a titer of 479.6 IU/mL at the first test and 405.2 IU/mL at the second test. As such, MLA method is a test system with high variability.

### Example 1: Quantification of antiserum to type A botulinum toxin by neutralization CMAP method

Standard type A botulinum antitoxin and the rabbit antiserum tested in Comparative Example by MLA method were diluted to 25, 12.5, 6.3 and 3.1 mIU/mL in physiological saline containing 0.5 w/v% of human serum albumin. Using the type A NTX as a test toxin, a test toxin dose was set to a dose with which amplitude at Day 1 of administration of a toxin alone is decreased to 1/3 of that before administration (10 LD50/mL). Standard type A botulinum antitoxin or the rabbit antiserum was mixed with a test toxin at an equivalent amount for reaction for 1 hour. The mixture (0.1 mL) was then administered intramuscularly to rats at the left gastrocnemius muscle.

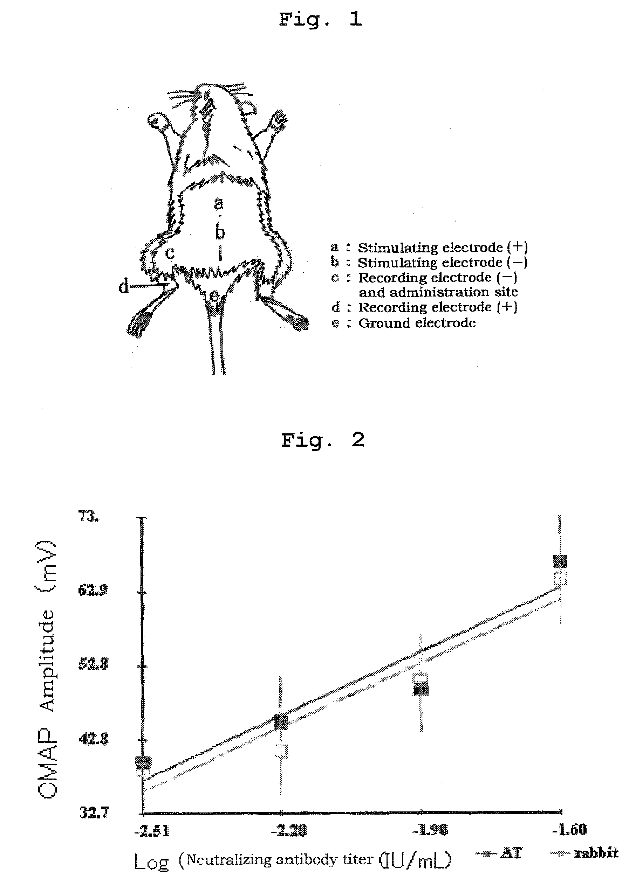

CMAP was measured by anesthetizing rats and, after verifying lost of eye-closure reflex, placing the animals prone. Arrangement of the electrodes were as follows: the stimulating electrode at the Radices spinales, the recording electrode(+) at the body of the gastrocnemius muscle of the hinder leg, the recording electrode(-) at the tendon of the gastrocnemius muscle of the hinder leg, and the ground electrode at the root of tail. Electric stimulus was applied at 25 mA for 0.2 msec. CMAP amplitudes after administration were measured in duplicate and a mean of the obtained CMAP amplitudes was used for data analysis. CMAP was measured before administration and a day after administration. Fig. 1 shows the administration site and the sites for measurement of CMAP.

Regression analysis was conducted using the amplitude data for the groups of the standard antitoxin and of the rabbit antiserum administered. The obtained regression lines were subjected to Parallel line assay method to determine an amount of the antitoxins. As a result, as shown in Fig. 2, both lines exhibited parallelism and linearity to reveal that they are substantially the same. As a consequence, it was proved that a titer obtained by the neutralization CMAP method was in conformity with a titer obtained by the mouse neutralization of MLA.

### Example 2: Quantification of antiserum to various types botulinum toxin by neutralization CMAP method

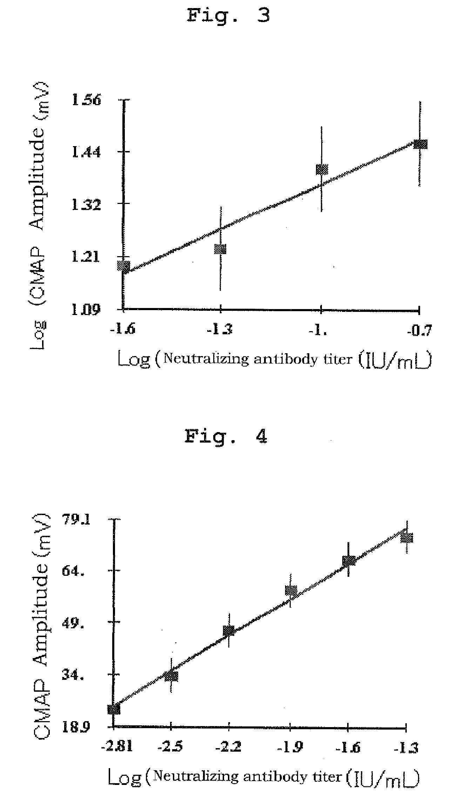

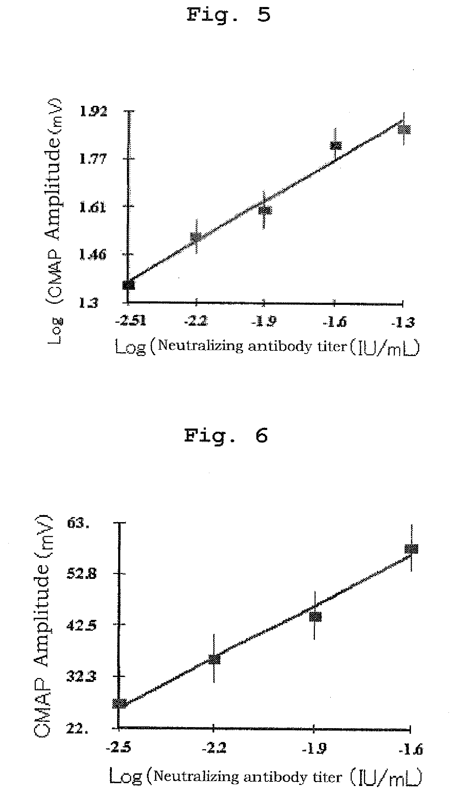

Each of standard types B, E and F botulinum antitoxins (given from National Institute of Infectious Diseases) were diluted to 200, 100, 50, 25, 12.5, 6.3, 3.1 and 1.5 mIU/mL. Using the various types NTXs prepared in Preparation as a test toxin, a test toxin dose was set to a dose with which amplitude at Day 1 of administration of a toxin alone is decreased to 1/3 of that before administration (120,000 LD50/mL for type B, 60 LD50/mL for type E, and 600 LD50/mL for type F). Each of the standard botulinum antitoxins was mixed with a test toxin at an equivalent amount for reaction for 1 hour. The mixture (0.1 mL) was then administered intramuscularly to rats at the left gastrocnemius muscle. A day after administration, CMAP was measured and regression analysis was conducted using the respective amplitude data. Dose response lines were obtained at 25-200 mIU/mL for type B (Fig. 3), at 1.5-50 mIU/mL for type E (Fig. 4) and at 3.1-50 mIU/mL for type F (Fig. 5).

### Example 3: Measurement of titer of neutralizing antibody in serum from patients who do not respond to therapy with botulinum toxin by neutralization CMAP method

Standard type A botulinum antitoxin was diluted to 25, 12.5, 6.25 and 3.1 mIU/mL. Sera from patients were used as they stand. Using the type A NTX as a test toxin, a test toxin dose was set to 10 LD50/mL. Standard type A botulinum antitoxin was mixed with a test toxin at an equivalent amount for reaction for 1 hour. The mixture (0.1 mL) was then administered intramuscularly to rats at the left gastrocnemius muscle. A day after administration, CMAP was measured and regression analysis was conducted using the amplitude data of the group of standard type A botulinum antitoxin administered. The obtained regression line was used as a calibration line (Fig. 6). An amount of the antitoxin was determined by applying the amplitude data from the group of patient serum to the calibration line. As a result, a titer of patient serum was found to be 5.1 mIU/mL (Table 1).

**Table 1**

| | Titer (mIU/mL) | CMAP amplitude (mV) |
|---|---|---|
| | 25 | 58.22 |
| Reference data | 12.5 | 44.53 |
| | 6.25 | 35.81 |
| | 3.13 | 26.83 |
| Sample | 5.08 | 30.84 |

### INDUSTRIAL APPLICABILITY

The method for quantification of a titer of a neutralizing antibody to a neurotoxin in accordance with the present invention may be utilized for (1) quantification of a neutralizing antibody to a neurotoxin, (2) quantification of antiserum to various types of botulinum toxin, and (3) measurement of a titer of a neutralizing antibody in serum from patients who receive therapy with a botulinum toxin but do not show a response thereto. In particular, the method provides a means for quantitatively measuring a titer of a neutralizing antibody to a Clostridium toxin with specificity and a high sensitivity.

## Claims

1. A method for quantification of a titer of a neutralizing antibody to a neurotoxin comprising the following steps:
(a) mixing a standard sample containing a fixed amount of a neurotoxin and a test sample containing a neutralizing antibody to said neurotoxin;
(b) administering the mixture obtained in step (a) into the muscle of a non-human mammal;
(c) several hours to several days after the administration, applying electric stimulus to the nerve which is dominant in the muscle that received the administration;
(d) measuring a compound muscle action potential (CMAP) due to contraction of the muscle of said mammal by application of electric stimulus with an electromyograph; and
(e) analyzing CMAP amplitude data obtained in step (d) for an extent of decrease in amplitude by the neurotoxin not neutralized, based on a dose dependent relationship between a titer of a neutralizing antibody and CMAP amplitude, to thereby quantify a titer of the neutralizing antibody contained in the test sample.

2. The method for quantification of claim 1 wherein the neurotoxin is selected from a neurotoxin produced by bacterium of Clostridium, a neurotoxin produced by fish or shellfish and a snake toxin.

3. The method for quantification of claim 2 wherein the bacterium of Clostridium is Clostridium botulinum.

4. The method for quantification of claim 3 wherein said Clostridium botulinum is selected from type A, B, C, D, E, F and G of Clostridium botulinum.

## Patentansprüche

1. Verfahren zum Quantifizieren eines Titers eines neutralisierenden Antikörpers, der gegen ein Neurotoxin gerichtet ist, wobei das Verfahren die folgenden Schritte umfasst:
(a) Mischen einer Standardprobe, die eine festgelegte Menge eines Neurotoxins enthält, mit einer Testprobe, die einen gegen das Neurotoxin gerichteten neutralisierenden Antikörper enthält;
(b) Verabreichen des in Schritt (a) erhaltenen Gemischs in den Muskel eines nicht-menschlichen Säugers;
(c) Einige Stunden bis einige Tage nach der Verabreichung, Setzung eines elektrischen Reizes an dem Nerv, der in dem Muskel, der die Verabreichung empfangen hat, dominant ist;
(d) Messen eines zusammengesetzten Muskelaktionspotenzials (CMAP) aufgrund der Kontraktion des Muskels des Säugers durch Setzen des elektrischen Reizes mit einem Elektromyographen; und
(e) Analysieren der CMAP-Amplitudendaten, die im Schritt (d) erhalten wurden, im Hinblick auf das Ausmaß der Abnahme der Amplitude durch das nicht neutralisierte Neurotoxin, basierend auf einer dosisabhängigen Beziehung zwischen einem Titer eines neutralisierenden Antikörpers und der CMAP-Amplitude, um dadurch einen Titer des neutralisierenden Antikörpers zu quantifizieren, der in der Testprobe enthalten ist.

2. Verfahren zum Quantifizieren nach Anspruch 1, wobei das Neurotoxin ausgewählt ist aus einem Neurotoxin, das von einem Clostridiumbakterium hergestellt ist, einem Neurotoxin, das von einem Fisch oder einem Schalentier hergestellt ist, und einem Schlangengift.

3. Verfahren zum Quantifizieren nach Anspruch 2, wobei das Clostridiumbakterium Clostridium botulinum ist.

4. Verfahren zum Quantifizieren nach Anspruch 3, wobei das Clostridium botulinum ausgewählt ist aus Typ A, B, C, D, E, F und G von Clostridium botulinum.

## Revendications

1. Méthode de quantification d'un titre d'un anticorps neutralisant dirigé contre une neurotoxine comprenant les étapes suivantes :
(a) le mélange d'un échantillon étalon contenant une quantité fixée d'une neurotoxine et d'un échantillon à tester contenant un anticorps neutralisant dirigé contre ladite neurotoxine ;
(b) l'administration du mélange obtenu dans l'étape (a) dans le muscle d'un mammifère non humain ;
(c) plusieurs heures à plusieurs jours après l'administration, l'application d'un stimulus électrique au nerf qui est dominant dans le muscle qui a reçu l'administration ;
(d) la mesure d'un potentiel d'action musculaire composé (PAMC) dû à la contraction du muscle dudit mammifère par application d'un stimulus électrique avec un électromyographe ; et
(e) l'analyse des données d'amplitude du PAMC obtenues dans l'étape (d) pour une étendue de diminution de l'amplitude par la neurotoxine non neutralisée, en se basant sur une relation dépendante de la dose entre un titre d'un anticorps neutralisant et une amplitude de PAMC, pour quantifier de cette façon un titre de l'anticorps neutralisant contenu dans l'échantillon à tester.

2. Méthode de quantification selon la revendication 1, dans laquelle la neurotoxine est choisie parmi une neurotoxine produite par la bactérie de Clostridium, une neurotoxine produite par un poisson ou un crustacé et une toxine de serpent.

3. Méthode de quantification selon la revendication 2, dans laquelle la bactérie de Clostridium est Clostridium botulinum.

4. Méthode de quantification selon la revendication 3, dans laquelle ledit Clostridium botulinum est choisi parmi le type A, B, C, D, E, F et G de Clostridium botulinum.
